(19) Europäisches Patentamt
European Patent Office
Office européen des brevets

(11) Publication number: **0 467 061 A2**

(12) **EUROPEAN PATENT APPLICATION**

(21) Application number: **91109237.7**

(51) Int. Cl.⁵: **C07C 51/14**

(22) Date of filing: **06.06.91**

(30) Priority: **18.06.90 JP 158999/90**

(43) Date of publication of application:
**22.01.92 Bulletin 92/04**

(84) Designated Contracting States:
**BE CH DE FR GB IT LI NL SE**

(71) Applicant: **IDEMITSU PETROCHEMICAL CO. LTD.**
**1-1, Marunouchi 3-chome Chiyoda-ku**
**Tokyo 100(JP)**

(72) Inventor: **Kawasaki, Hiroshi, c/o IDEMITSU PETROCHEM. CO.,LTD**
**No. 1-1, Shingu-cho**
**Tokuyama-shi, Yamaguchi-ken(JP)**
Inventor: **Tanibayashi, Katsunori, c/o IDEMITSU PETROCHEMICAL CO., LTD., No. 1-1, Shingu-cho**
**Tokuyama-shi, Yamaguchi-ken(JP)**
Inventor: **Kato, Tadashi, c/o IDEMITSU PETROCHEMICAL CO., LTD**
**No. 1-1, Shingu-cho**
**Tokuyama-shi, Yamaguchi-ken(JP)**

(74) Representative: **Türk, Gille, Hrabal**
**Brucknerstrasse 20**
**W-4000 Düsseldorf 13(DE)**

(54) **Process for production of carboxylic acid.**

(57) The present invention relates to a process for producing carboxylic acid which comprises extracting a carbonization reaction product obtained by adding carbon monoxide and water to olefin in the presence of a catalyst selected from the group consisting of a catalyst comprising an aqueous sulfuric acid solution, a catalyst comprising an aqueous sulfuric acid solution and phosphoric acid, a catalyst comprising an aqueous sulfuric acid solution and metal oxide, and a catalyst comprising an aqueous sulfuric acid solution, phosphoric acid and metal oxide to thereby obtain carboxylic acid, and withdrawing a portion of the catalyst, diluting the catalyst withdrawn with water, extracting the diluted catalyst and returning the thus regenerated catalyst to the reaction system.

In accordance with the process of the present invention, high purity carboxylic acid is produced by efficiently regenerating the catalyst for reuse.

In accordance with the process of the present invention, the catalyst is efficiently regenerated, recovered, and returned to the reaction system for reuse; therefore the catalyst is effectively utilized. Thus the catalyst cost can be reduced.

Moreover, since the activity of the catalyst is fully maintained, the carbonization reaction can be carried out in a stabilized manner.

Moreover, since side reactions can be prevented, high purity carboxylic acid can be produced with high efficiency and at low production costs.

EP 0 467 061 A2

*FIG. 1*

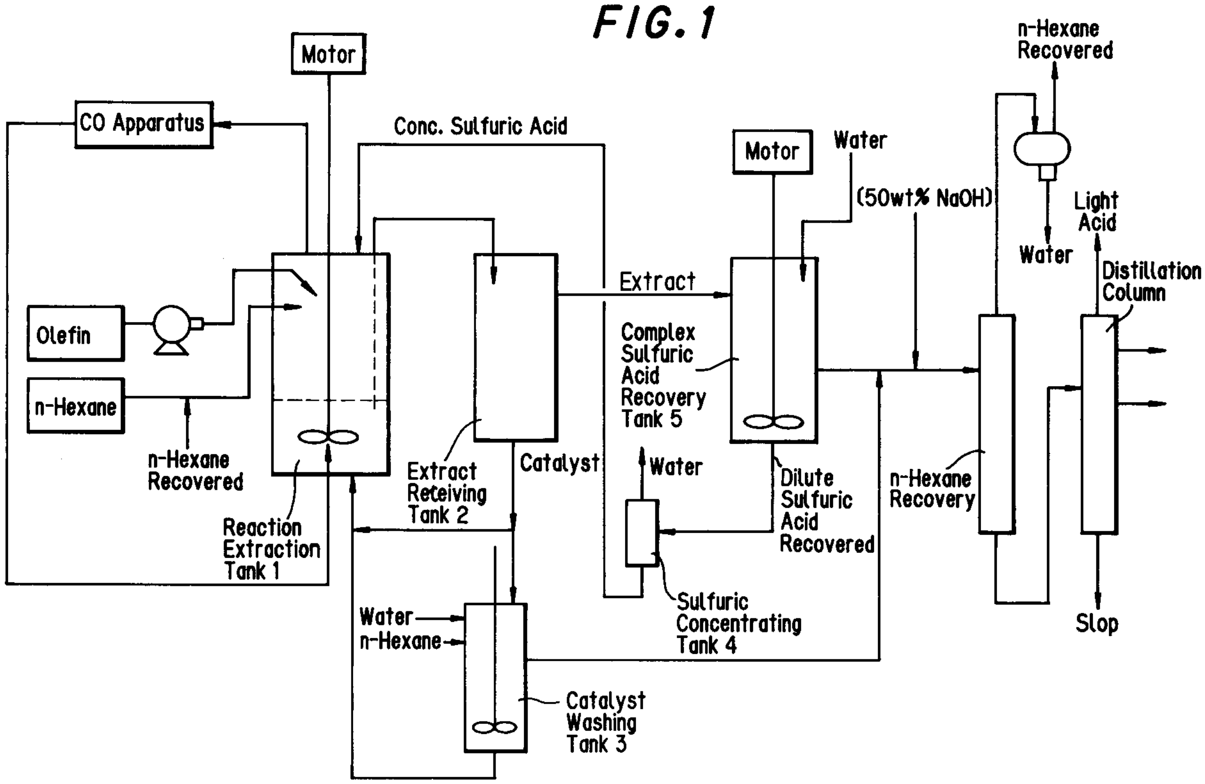
Motor
CO Apparatus
Conc. Sulfuric Acid
Motor
Water
(50wt% NaOH)
n-Hexane Recovered
Light Acid
Water
Distillation Column
Olefin
n-Hexane
Extract
Complex Sulfuric Acid Recovery Tank 5
n-Hexane Recovered
Reaction Extraction Tank 1
Extract Receiving Tank 2
Catalyst
Water
Dilute Sulfuric Acid Recovered
n-Hexane Recovery
Sulfuric Concentrating Tank 4
Slop
Water
n-Hexane
Catalyst Washing Tank 3

## BACKGROUND OF THE INVENTION

The present invention relates to a process for production of carboxylic acid which is used as paint material, polymerization catalyst, additive for resin, solvent, wood preservative, and so forth. More particularly the present invention is concerned with a process for producing high purity carboxylic acid in which a catalyst is efficiently regenerated and thus the catalyst can be effectively utilized. And also, the present invention is concerned with a process for producing high purity carboxylic acid in which the regenerated catalyst thus obtained maintains high activity, the carbonization reaction can be carried out in a stabilized manner, and side reaction can be prevented.

Further the present invention is concerned with a process for producing the carboxylic acid at low production costs.

Moreover the present invention relates to a process for efficiently producing a carboxylic acid alkali salt from the carboxylic acid obtained above.

It is known as described in Japanese Patent Publication Nos. 20530/1973, 35055/1973, 3511/1974, etc. that tertiary carboxylic acid is obtained by reacting carbon monoxide and water with olefin, alcohol or hydrocarbon in a strong acid.

In practice of these methods on a commercial scale, it is important that a catalyst solution is repeatedly used without reduction of its catalytic activity, because sulfuric acid in the catalyst solution is used in such a large amount as at least 3 moles per mole of C = C bond of the olefin. Moreover, disposal of the used catalyst is undesirable from a viewpoint of preventing pollution and from an economic standpoint.

It has been strongly desired, therefore, to develop a method in which a catalyst solution can be repeatedly used without reduction of its catalytic activity.

However the catalyst solution (acid solution), once diluted with water, loses its catalytic activity. If, therefore, the total catalyst solution is concentrated and recovered, the concentration cost is seriously increased. Moreover a problem arises in that monovalent copper ions present in the acid solution are subject to oxidiation or disproportionation in the course of concentration by heating, thereby losing its catalytic activity.

Japanese Patent Application Laid-Open No. 123614/1975 proposes a method in which the catalyst solution is continuously used by continuously extracting the reaction product with an organic solvent. Usually, however, the extract from the catalyst solution contains sulfuric acid in the form of a complex in an amount proportional to the number of moles of the extracted carboxylic acid.

The above method, therefore, has a disadvantage in that unless the complex sulfuric acid is recovered and reused by returning to the reaction system, or the corresponding amount of sulfuric acid is supplied, the amount of sulfuric acid in the catalyst is decreased, and carbonization activity cannot be maintained. Usually when olefin is carbonized, the resulting carbonyl compound is immediately hydrolyzed by water in the catalyst, leading to formation of carboxylic acid. Thus the method has another disadvantage in that unless water consumed in the reaction (theoretical amount of water) is added after the completion of the reaction, the sulfuric acid concentration cannot be maintained, resulting in a reduction of the catalytic activity, and thus the reaction cannot be performed in a stabilized manner.

Thus the present applicant has proposed in Japanese Patent Application Laid-Open No. 76434/1986 a method in which an extract obtained by extraction of the reaction product with an organic solvent is washed with a theoretical amount of water to recover sulfuric acid, and the sulfuric acid thus recovered is returned to the reaction system for its reuse.

In accordance with this method, the catalyst can be used repeatedly more than 50 times. This method, however, still has problems that in repeated use of the catalyst, unextracted carboxylic acid, by-produced neutral oil, sulfur compounds, etc. are accumulated in the catalyst, resulting in a gradual reduction of the catalytic activity.

## SUMMARY OF THE INVENTION

It has now been found according to the present invention that by withdrawing a portion of a catalyst from a reaction product resulitng from a carbonization reaction while at the same time obtaining carboxylic acid by extraction, diluting the above withdrawn catalyst with water, again applying an extraction treatment, and returning the regenerated catalyst thus obtained to the reaction system for its reuse, the unextracted portion of the catalyst can be reduced and the catalyst can be maintained at high activity, as a result of which the catalyst cost can be greatly decreased.

The present invention provides a process for producing carboxylic acid which comprises extracting a carbonization reaction product obtained by adding carbon monoxide and water to olefin in the presence of a

catalyst selected from the group consisting of a catalyst comprising an aqueous sulfuric acid solution, a catalyst comprising an aqueous sulfuric acid solution and phosphoric acid, a catalyst comprising an aqueous sulfuric acid and metal oxide, and a catalyst comprising an aqueous sulfuric acid, phosphoric acid and metal oxide to thereby obtain carboxylic acid, and withdrawing a portion of the catalyst, diluting the catalyst withdrawn with water, extracting the diluted catalyst, and returning the regenerated catalyst thus obtained to the reaction system.

The present invention further provides a process for producing a carboxylic acid alkali salt which comprising adding an alkali metal hydroxide to carboxylic acid obtained by the above method.

## BRIEF DESCRIPTION OF THE DRAWING

Fig. 1 is a flow diagram illustrating an embodiment of the process of the present invention;

Fig. 2 is a graph showing a relation between a rate of extraction and a sulfuric acid concentration in each component of the reaction product;

Fig. 3 is a graph showing a relation between an oil content of the catalyst before the reaction and the number of repeated use of the catalyst;

Fig. 4 is a graph showing a relation between an oil content of the catalyst and the number of repeated use of the catalyst, after completion of the reaction; and

Fig. 5 is a graph showing a relation between a sulfur content of the reaction product (reaction mixture)-and the number of repeated use of the catalyst.

## DETAILED DESCRIPTION OF THE INVENTION

The process of the present invention will hereinafter be explained with reference to the accompanying drawing wherein Fig. 1 is a flow diagram of an embodiment of the present process. In Fig. 1, reference numeral 1 indicates a reaction-extraction tank; reference numeral 2 indicates an extract receiving tank; reference numeral 3 indicates a catalyst washing tank; reference numeral 4 indicates a sulfuric acid concentrating tank; and reference numeral 5 indicates a complex sulfuric acid recovery tank.

The reaction-extraction tank 1 is charged with starting materials (olefin, carbon monoxide and water) and a catalyst, and a carbonization reaction is carried out therein.

The olefin starting material to be used in the process of the present invention is not always critical as long as it is monoolefin. In industrial practice, a mixture of polyolefins having an isobutylene unit content of at least 60% by weight, which is available at relatively low costs, is preferably used.

Carbon monoxide is most preferred to be pure, but carbon monoxide-containing gas such as water gas, producer gas or coke oven gas can also be used.

Pure water is preferably used; for example, distilled water, ion exchanged water, etc. are suitably used.

The catalyst to be used in the process of the present invention is selected from a group consisting of a catalyst comprising an aqueous sulfuric acid solution, a catalyst comprising an aqueous sulfuric acid solution and phosphoric acid, a catalyst comprising an aqueous sulfuric acid solution and metal oxide, and a catalyst comprising an aqueous sulfuric acid solution, phosphoric acid and metal oxide. Of these catalysts, the catalyst comprising an aqueous sulfuric acid solution and metal oxide and the catalyst comprising an aqueous sulfuric acid solution, phosphoric acid and metal oxide are preferably used.

In accordance with the process of the present invention, a carbonization reaction is carried out using the aforementioned starting materials and catalyst.

In connection with reaction conditions, a reaction temperature is usually -10 °C to 80 °C and preferably 0 to 30 °C, a reaction pressure is usually 0 to 100 $kg/cm^2G$ and preferably 2 to 50 $kg/cm^2G$, and a carbon monoxide partial pressure is at least 0.1 $kg/cm^2G$. In addition, a reaction time is usually sufficient to be 10 to 120 minutes after completion of addition of olefin.

When olefin, carbon monoxide and water are brought into contact with each other under the conditions as described above, a carbon skeleton of the olefin starting material is subject to acid-catalyzed skeleton rearrangement, resulting in formation of a mixture of tertiary carboxylic acids having one more carbon atoms than the olefin starting material. In addition, carboxylic acids having various numbers of carbon atoms are formed as a result of cleavage and recombination of an intermediate product of carbocation.

In accordance with the process of the present invention, the carbonization reaction product is first subjected to an extraction treatment. More specifically an extraction solvent is supplied to the reaction-extraction tank 1 where the carbonization reaction has been carried out, and the extraction treatment is performed.

Extraction solvents that can be used include aliphatic hydrocarbons such as pentane, hexane, cyclohexane, heptane and octane; aromatic hydrocarbons such as benzene, toluene, and xylene; and halogenated hydrocarbons such as dichloromethane, chloroform, carbon tecrachloride, trichloroethylene, and trichloroethane. In this extraction treatment, the carboxylic acid formed and sulfuric acid are extracted.

Of the above extraction solvents, the halogenated hydrocarbons are not preferred because they are expensive, are large in dissolution loss when dissolved in water, are easily decomposed at the time of recovery, and are toxic, although they are excellent in extraction. The aromatic hydrocarbons are also not preferred because they are subject to partial nuclear alkylation by the olefin during the presence of the catalyst, although they have a large extraction power.

Thus the aliphatic hydrocarbons are preferred because, although inferior in the extraction power to the above halogenated hydrocarbons and aromatic hydrocarbons, they are unexpensive, of low toxicity and chemically stable. In particular, hexane is suitably used.

The amount of the extraction solvent used is 0.1 to 5.0 times (by volume), preferably 0.2 to 2.0 times (by volume) the volume of the reaction mixture.

If the amount of the extraction solvent used is less than 0.1 time (by volume) the volume of the reaction mixture, the rate of extraction is decreased, and if it is more than 5.0 times the volume of the reaction mixture, a solvent recovery cost is increased.

The reaction mixture with the extraction solvent added thereto is separated by allowing to stand. Nearly all of the extract and a portion of the catalyst solution are sent to the extract-receiving tank 2.

In this extract, sulfuric acid in the amount in proportional to the number of moles of the extracted carboxylic acid is contained in the form of complex sulfuric acid. For this reason, water is added to the extract in the complex sulfuric acid recovery tank 5 where the extract is washed with water and separated into an oil layer containing carboxylic acid and an aqueous layer containing sulfuric acid (dilute sulfuric acid).

In this manner, the objective carboxylic acid is obtained, and sulfuric acid is separated and recovered as dilute sulfuric acid.

If necessary, the dilute sulfuric acid thus obtained may be separated from waste water and concentrated in the sulfuric concentrating tank 4, and then sent to the reaction-extraction tank 1.

In connection with the amount of water added for separation and recovery of sulfuric acid, if water is added in an amount of 0.3 to 3 times the amount of water consumed in the carbonization reaction (theoretical water amount), the complex sulfuric acid can be recovered almost completely. When, however, the dilute sulfuric acid recovered is concentrated and reused, or disposed without recovery, the amount of water added may be in excess of 3 times the theoretical water amount.

Theoretically, if the complex sulfuric acid is mixed and washed with the theoretical amount of water, and the dilute sulfuric acid recovered is returned to the catalyst system as it is, the concentration of sulfuric acid will not change because a material balance between sulfuric acid and water is kept constant and, therefore, the catalyst will be able to repeatedly use permanently. In practice, however, carboxylic acid and by-products are accumulated in the catalyst, leading to a gradual reduction of carbonization activity.

That is, in the catalyst as a lower layer in the reaction-extraction tank 1, light fatty acids such as pivalic acid and by-products such as sulfur compounds are gradually accumulated as a result of repeated use, and thus the catalyst is deteriorated. In order to prevent this catalyst deterioration, it is necessary to remove the oil components from the catalyst as much as possible.

As a result of detailed examination on a rate of extraction of each component when extracted with aliphatic hydrocarbon such as n-hexane, it has been found that although the aliphatic hydrocarbon (e.g., n-hexane) itself is inferior in the extracting power, if water is added to thereby decrease the concentration of sulfuric acid in the catalyst, more specifically $(H_2SO_4/(H_2SO_4 + H_2O)\} \times 100$ (wt%) to not more than 80%, the aliphatic hydrocarbon can extract and remove the oil components with high efficiency.

Catalysts having various sulfuric acid concentrations were prepared by adding a predetermined amount of water to 108.0 g of a strong acid mixture, as a catalyst, consisting of 65.1% by weight of sulfuric acid, 28.4 by weight of phosphoric acid, and 6.5% by weight of water (in this case, the sulfuric acid concentration according to the above definition was 90.9% by weight), and extracted with 26.6 g of n-hexane. The rate of extraction of each component in 18.15 g (92.0 mmol) of a carboxylic acid mixture is shown in Fig. 2. From the results shown in Fig. 2, it has been found that if the concentration of sulfuric acid in the catalyst is decreased to not more than 80%, the oil component (indicating all organic compounds in the catalyst, i.e., carboxylic acid and by-products) can be extracted and removed with efficiency. Fig. 2 is a graph showing a relation between the rate of extraction of each component in the reaction product and the sulfuric acid concentration.

However, once the total catalyst is diluted with water, it is difficult to increase the sulfuric acid concentration until the carbonization activity is regained.

Thus it has been found that if a portion of the catalyst is withdrawn from the reaction-extraction tank 1, diluted with water and then again extracted with a solvent, and the regenerated catalyst almost freed of the oil component by the re-extraction is returned to the reaction-extraction tank 1, accumulation of the oil component in the catalyst can be prevented and the carbonization reaction can be carried out in a stabilized manner.

In accordance with the process of the present invention, the catalyst is regenerated as follows.

A portion of the catalyst containing light components such as pivalic acid is withdrawn from the lower layer (aqueous layer) in the reaction-extraction tank 1, sent to the extract receiving tank 2 and then introduced into the catalyst washing tank 3 where the catalyst is diluted with water, and then re-extraction of the carboxylic acid component and regeneration of the catalyst are carried out by adding the extraction solvent. The residual aqueous layer is supplied to the subsequent reaction as it is.

The amount of the catalyst withdrawn varies with the amount of water added to the catalyst and the concentration of concentrated sulfuric acid supplied, and can be suitably chosen from the range of 5 to 30% by weight of the total catalyst amount.

The amount of water used to dilute the catalyst should be not more than the amount of water consumed per one batch (theoretical water amount). That is, it is controlled taking into consideration the amount of the catalyst withdrawn so that the concentration of sulfuric acid in the aqueous solution is 70 to 85%.

The solvent for use in the re-extraction may be the same as or different from the solvent used for the first extraction, and can be suitably chosen from the aforementioned solvents. The amount of the solvent used varies with the amount of the catalyst withdrawn; it is usually used in such an amount that the volume ratio of solvent to catalyst (solvent/catalyst) is 0.1/1 to 50/1.

The carboxylic acid thus obtained by the re-extraction is introduced from the extract receiving tank 2 directly into the complex sulfuric acid recovery tank 5 where the complex sulfuric acid is recovered. Then it may be combined with the carboxylic acid extract, if necessary, or they may be subjected to posttreatment, separately. In any way, the desired product is obtained by purifying by means of distillation, for example.

The regenerated catalyst regenerated in the catalyst washing tank 3 is combined with the catalyst which is returned from the extract receiving tank 2 to the reaction-extraction tank 1 as it is, and then returned to the reaction-extraction tank 1 for reuse.

In accordance with the process of the present invention, high purity carboxylic acid is produced by efficiently regenerating the catalyst for reuse.

The carboxylic acid obtained by the process of the present invention is, for example, a mixture of aliphatic tertiary carboxylic acids having 5 to 17 carbon atoms.

Addition of an alkali metal hydroxide to the above carboxylic acid results an alkali metal salt of carboxylic acid.

In accordance with the process of the present invention, the catalyst is efficiently regenerated, recovered, and returned to the reaction system for reuse; therefore the catalyst is effectively utilized. Thus the catalyst cost can be reduced.

Moreover, since the activity of the catalyst is fully maintained, the carbonization reaction can be carried out in a stabilized manner.

Moreover, since side reactions can be prevented, high purity carboxylic acid can be produced with high efficiency and at low production costs.

The present invention is described in greater detail with reference to the following examples. Example

According to the flow diagram shown in Fig. 1, the reaction was carried out through the following four steps.

First Step

540 g of a strong acid mixture consisting of 65.1% by weight of sulfuric acid, 28.4% by weight of phosphoric acid and 6.5% by weight of water, and 13.2 g of cuprous oxide were introduced into the reaction-extraction tank 1 (1-liter electromagnetic induction stirring type autoclave having an inner diameter of 8 cm), where they were stirred at 15°C under a carbon monoxide pressure of 15 $kg/cm^2G$ for 16 hours to dissolve the cuprous oxide, thereby preparing a catalyst solution. As illustrated by the reaction formula shown below, the cuprous oxide absorbed carbon monoxide, becoming a cupper carbonyl complex.

$$Cu_2O + 2H_2SO_4 + 6CO \rightarrow 2Cu^{+}(CO)_3HSO_4^{\ominus} + H_2O$$

Second Step

Carbon monoxide was blown into the catalyst solution at a rate of 13.7 N1/hr, and while stirring at 1,300 rpm, $C_{12}$ polyolefin (trade name: IP1620R produced by Idemitsu Petrochemical Co., Ltd.) was supplied over 90 minutes. After completion of the supply of polyolefin, stirring was continued for 60 minutes to cause the reaction.

Third Step

After completion of the reaction, the reaction mixture was allowed to stand and the pressure was returned to atmospheric pressure. Then 200 ml of n-hexane was added, and the resulting mixture was stirred at 1,300 rpm for 20 minutes to extract the reaction mixture with the n-hexane. Almost all of the extract and a portion of the catalyst solution were introduced into the extract receiving tank 2, and then the extract was introduced into the complex sulfuric acid recovery tank 5. The extract was washed with 8.27 g of water to obtain a carboxylic acid component and at the same time, recover dilute sulfuric acid. This dilute sulfuric acid can be concentrated in the sulfuric (acid) concentrating tank 4 and reused.

Fourth Step

From the catalyst layer introduced from the reaction-extraction tank 1 into the extract receiving tank 2, 64 g of the catalyst was withdrawn, and it was then introduced into the catalyst washing tank 3. The residual catalyst was returned to the reaction-extraction tank 1 as it is and subjected to reutilization. To 64 g of the catalyst in the catalyst washing tank 3 was added 8.27 g of water to dilute the catalyst with water, and then the diluted catalyst was extracted once with 25 ml of n-hexane. The catalyst layer after the extraction was returned to the reaction-extraction tank 1. A carboxylic acid component in the resulting n-hexane layer was mixed with the carboxylic acid component in the above obtained n-hexane layer, and recovered. In order to neutralize small amounts of sulfur compounds, a small amount of a 50 wt% aqueous sodium hydroxide solution was added, and then the n-hexane was recovered to obtain a crude product. Further this crude product was distilled to obtain a carboxylic acid product.

The operation of the above first to fourth steps was repeated 21 times.

The reaction was stable for all batches; the reaction results were nearly the same for all batches.

A carboxylic acid yield, a complex sulfuric acid/carboxylic acid ratio, and a sulfur content of the reaction mixture were as follows.

Carboxylic acid yield: 100 to 110 mol%
(carboxylic acid yield = number of moles of the extracted carboxylic acid per mole of the olefin starting material) Complex sulfuric acid/carboxylic acid ratio: 0.40 to 0.50 (equivalent ratio)
Sulfur content of the reaction mixture: less than 400 wt ppm

Table 1 shows a composition of the extract (17th batch); Table 2 shows a composition of the catalyst before the carbonization reaction (i.e., catalyst composition in the state that the catalyst composition is stabilized); Table 3 shows a composition of oil component in the catalyst of Table 2; and Table 4 shows a rate of extraction of each component and a rate of extraction of the whole.

In addition, a relation between oil components in the catalyst before the reaction and the number of repeated use of the catalyst is shown in Fig. 3.

Table 1

| Composition of Extract (excluding extraction solvent) (wt%) | | |
|---|---|---|
| | Extract | Extract after Re-extraction |
| Neutral oil | 2.0 | 0.0 |
| Pivalic acid | 2.5 | 29.2 |
| $C_6$ acid | 1.5 | 12.3 |
| $C_{7,8}$ acid | 2.4 | 10.4 |
| $C_9$ acid | 8.8 | 17.2 |
| $C_{10}$ to $C_{13}$ acid | 82.8 | 29.1 |
| $C_{14}^{+(*)}$ | 0.0 | 1.8 |
| Total | 100.0 | 100.0 |

(*) Containing sulfur compounds.

Table 2

| Composition of Catalyst before Carbonization Reaction (wt%) | |
|---|---|
| $(CuCO)HSO_4$ | 4.8 |
| Oil fraction | 9.7 |
| $H_2SO_4$ | 55.7 |
| $H_3PO_4$ | 22.2 |
| $H_2O$ | 7.6 |
| Total | 100.0 |

Table 3

| Composition of Oil in Catalyst (wt%) | |
|---|---|
| Neutral oil | 6.4 |
| Pivalic acid | 28.7 |
| $C_6$ acid | 11.7 |
| $C_{7,8}$ acid | 10.1 |
| $C_9$ acid | 21.8 |
| $C_{10}$ to $C_{13}$ acid | 17.4 |
| $C_{14}^{+(*)}$ | 3.9 |
| Total | 100.0 |

(*) Containing sulfur compounds;

Table 4

Rate of Extraction of Each Component and Rate of Extraction of Whole Product (wt%)

| | Extract | Extract after Re-extraction | Total |
|---|---|---|---|
| Neutral Oil | 33.8 | - | 33.8 |
| Pivalic acid ($C_5$) | 11.4 | 8.3 | 19.7 |
| $C_6$ acid | 15.8 | 8.1 | 23.9 |
| $C_{7,8}$ acid | 25.4 | 6.9 | 32.3 |
| $C_9$ acid | 37.0 | 4.5 | 41.5 |
| $C_{10}$ to $C_{13}$ acid | 85.9 | 1.9 | 87.8 |
| $C_{14}$+(*) | - | 4.5 | 4.5 |
| Total | 60.0 | 3.7 | 63.7 |

(*) Containing sulfur compounds.

Comparative Example 1

The procedure of Example was followed, but at 23 to 36th batches, the operation of the fourth step was omitted, and the recovered dilute sulfuric acid obtained at the third step was recycled to the catalyst system to use the catalyst repeatedly.

In connection with reaction results at each batch, (1) the concentration of oil in the catalyst was increased, (2) the sulfur content of the crude product was increased, (3) the selectivity of $C_{10}$ to $C_{13}$ was decreased, and (4) the amounts of neutral oil and sulfur compounds in the catalyst were increased.

The yield of carboxylic acid, the complex sulfuric acid/carboxylic acid ratio, and the sulfur content of the reaction mixture were as follows.

Carboxylic acid yield: 100 to 110 mol%

(carboxylic acid yield = number of moles of extracted carboxylic acid per mole of the olefin starting material) Complex sulfuric acid/carboxylic acid ratio: 0.40/1 to 0.50/1 (equivalent ratio)

Sulfur content of the reaction mixture: 500 to 700 wt ppm

As the batch number increased, the sulfur content tended to increase.

Table 5 shows a composition of the extract (at the 36th batch); Table 6 shows a composition of the catalyst before the carbonization reaction (catalyst composition before the reaction at the 36th batch); and Table 7 indicates a rate of extraction of each component and a rate of extraction of the whole. In addition, a relation between the oil content of the catalyst before the reaction and the number of repeated use of the catalyst is shown in Fig. 3.

Table 5

| Composition of Extract (excluding the extraction solvent) (wt%) | |
|---|---|
| | Extract |
| Neutral Oil | 2.8 |
| Pivalic acid | 4.2 |
| $C_6$ acid | 2.2 |
| $C_{7,8}$ acid | 3.3 |
| $C_9$ acid | 9.5 |
| $C_{10}$ to $C_{13}$ acid | 75.3 |
| $C_{14}{}^{+(*)}$ | 2.7 |
| Total | 100.0 |

(*) Containing sulfur compounds.

Table 6

| Composition of Catalyst before Carbonization Reaction (wt%) | |
|---|---|
| $(CuCO)HSO_4$ | 4.1 |
| Oil fraction | 16.8 |
| $H_2SO_4$ | 50.3 |
| $H_3PO_4$ | 21.4 |
| $H_2O$ | 7.4 |
| Total | 100.0 |

Table 7

| Rate of Extraction of Each Component and Rate of Extraction of the Whole (wt%) | |
|---|---|
| | Extract |
| Neutral Oil | 19.9 |
| Pivalic acid | 12.7 |
| $C_6$ acid | 13.7 |
| $C_{7,8}$ acid | 24.6 |
| $C_9$ acid | 28.3 |
| $C_{10}$ to $C_{13}$ acid | 78.7 |
| $C_{14}{}^{+(*)}$ | 26.1 |
| Total | 46.3 |

(*) Containing sulfur compounds.

As can be seen from the above results, if the re-extraction operation is omitted, the rate of extraction of light carboxylic acid such as pivalic acid and $C_6$ acid is decreased, and under influences of the decrease in the rate of extraction of the light carboxylic acid, the rate of extraction of $C_7$ or more carboxylic acid is decreased. Thus, the oil content of the catalyst is increased, accompanying by an increase of side reactions.

Comparative Example 2

The procedure of Example 1 was repeated with the exception that the re-extraction was omitted.

The oil content of the catalyst was increased to 23% by weight. At this time, the volume of the catalyst was increased to about 1.6 times. In addition, the sulfur content of the reaction mixture was increased batch by batch.

A relation between the oil content of the catalyst and the number of repeated use of the catalyst, after the completion of the reaction is shown in Fig. 4. In addition, a relation between the sulfur content of the reaction mixture and the number of repeated use of the catalyst is shown in Fig. 5.

## Claims

1. A process for producing carboxylic acid which comprises extracting a carbonization reaction product obtained by adding carbon monoxide and water to olefin in the presence of a catalyst selected from the group consisting of a catalyst comprising an aqueous sulfuric acid solution, a catalyst comprising an aqueous sulfuric acid solution and phosphoric acid, a catalyst comprising an aqueous sulfuric acid solution and metal oxide, and a catalyst comprising an aqueous sulfuric acid solution, phosphoric acid and metal oxide to thereby obtain carboxylic acid, and withdrawing a portion of the catalyst, diluting the catalyst withdrawn with water, extracting the diluted catalyst and returning the thus regenerated.

2. The process as claimed in Claim 1 wherein the olefin is a mixture of polyolefins having an isobutylene unit content of at least 60% by weight.

3. The process as claimed in Claim 1 wherein the carbonization reaction is carried out under a reaction temperature of -10° C to 80° C, a reaction pressure of 0 to 100 $kg/cm^2G$, and a carbon monoxide partial pressure of at least 0.1 $kg/cm^2G$.

4. The process as claimed in Claim 1, wherein a solvent for the extraction is aliphatic hydrocarbon

5. The process as claimed in Claim 1 wherein an amount of a solvent for the extraction is 0.1 to 5.0 times (by volume) the volume of the reaction mixture.

6. The process as claimed in Claim 1 wherein an amount of the catalyst withdrawn is in the range of 5 to 30% by weight of the total catalyst amount.

7. A process for producing an alkali salt of carboxylic acid which comprises adding an alkali metal hydroxide to the carboxylic acid obtained by the process of Claim 1.

*F/G. 1*

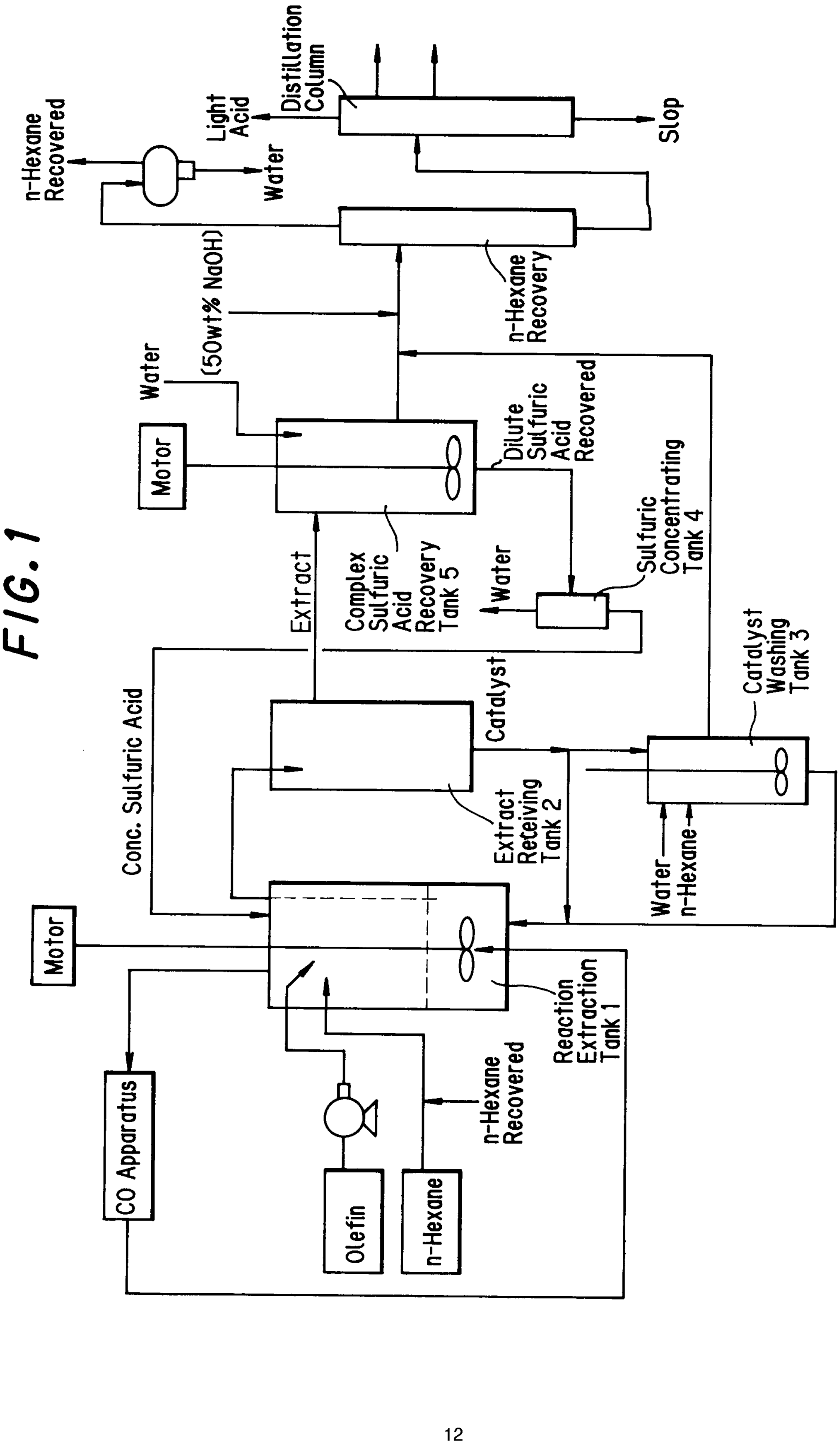
n-Hexane Recovered
Light Acid
Distillation Column
Water
Slop
(50wt% NaOH)
n-Hexane Recovery
Water
Motor
Dilute Sulfuric Acid Recovered
Extract
Complex Sulfuric Acid Recovery Tank 5
Water
Sulfuric Concentrating Tank 4
Conc. Sulfuric Acid
Catalyst
Catalyst Washing Tank 3
Extract Reteiving Tank 2
Water
n-Hexane
Motor
Reaction Extraction Tank 1
CO Apparatus
n-Hexane Recovered
Olefin
n-Hexane

FIG.2

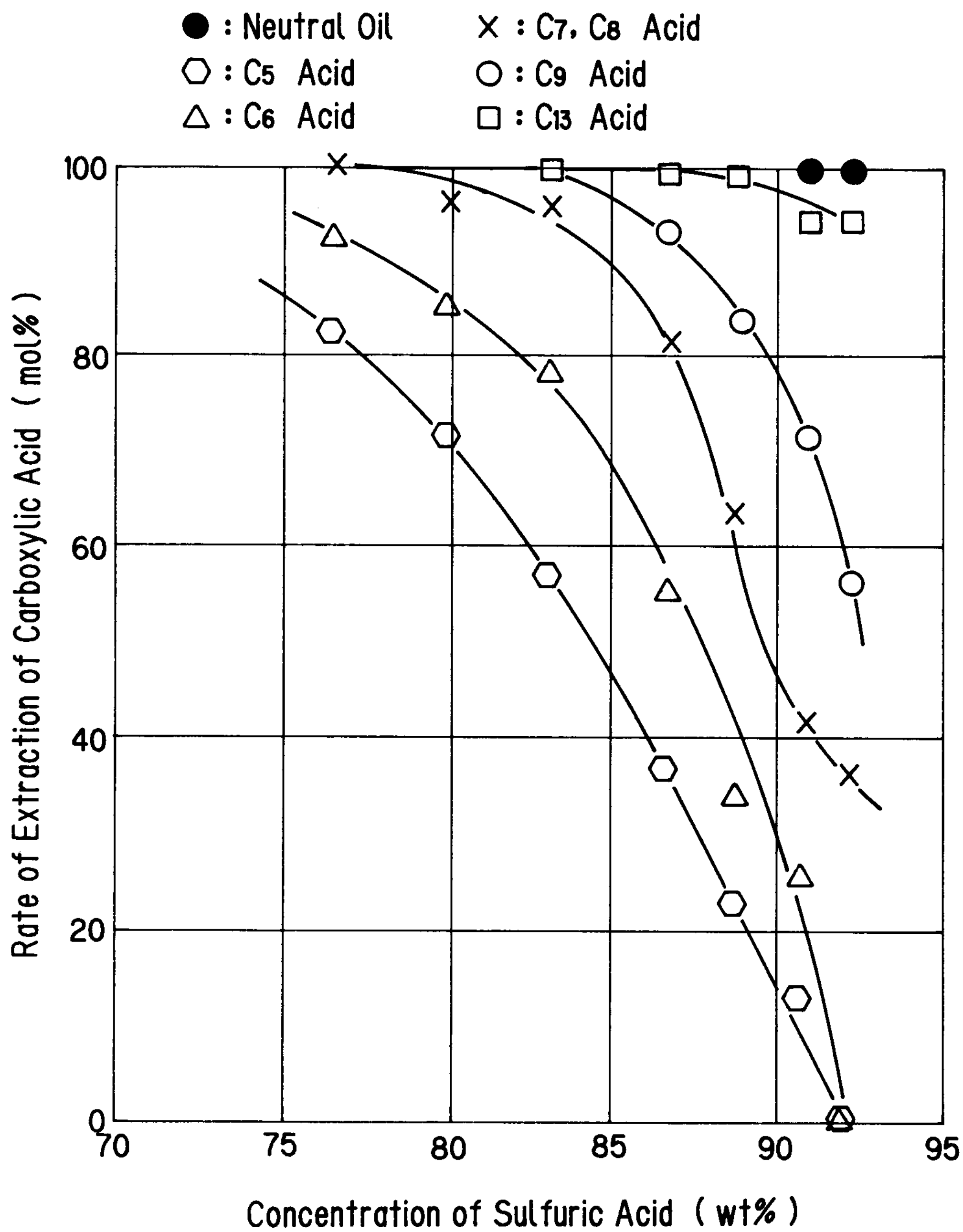
● : Neutral Oil
× : C7, C8 Acid
⬡ : C5 Acid
○ : C9 Acid
△ : C6 Acid
□ : C13 Acid
Rate of Extraction of Carboxylic Acid ( mol% )
0
20
40
60
80
100
70
75
80
85
90
95
Concentration of Sulfuric Acid ( wt% )

FIG. 3

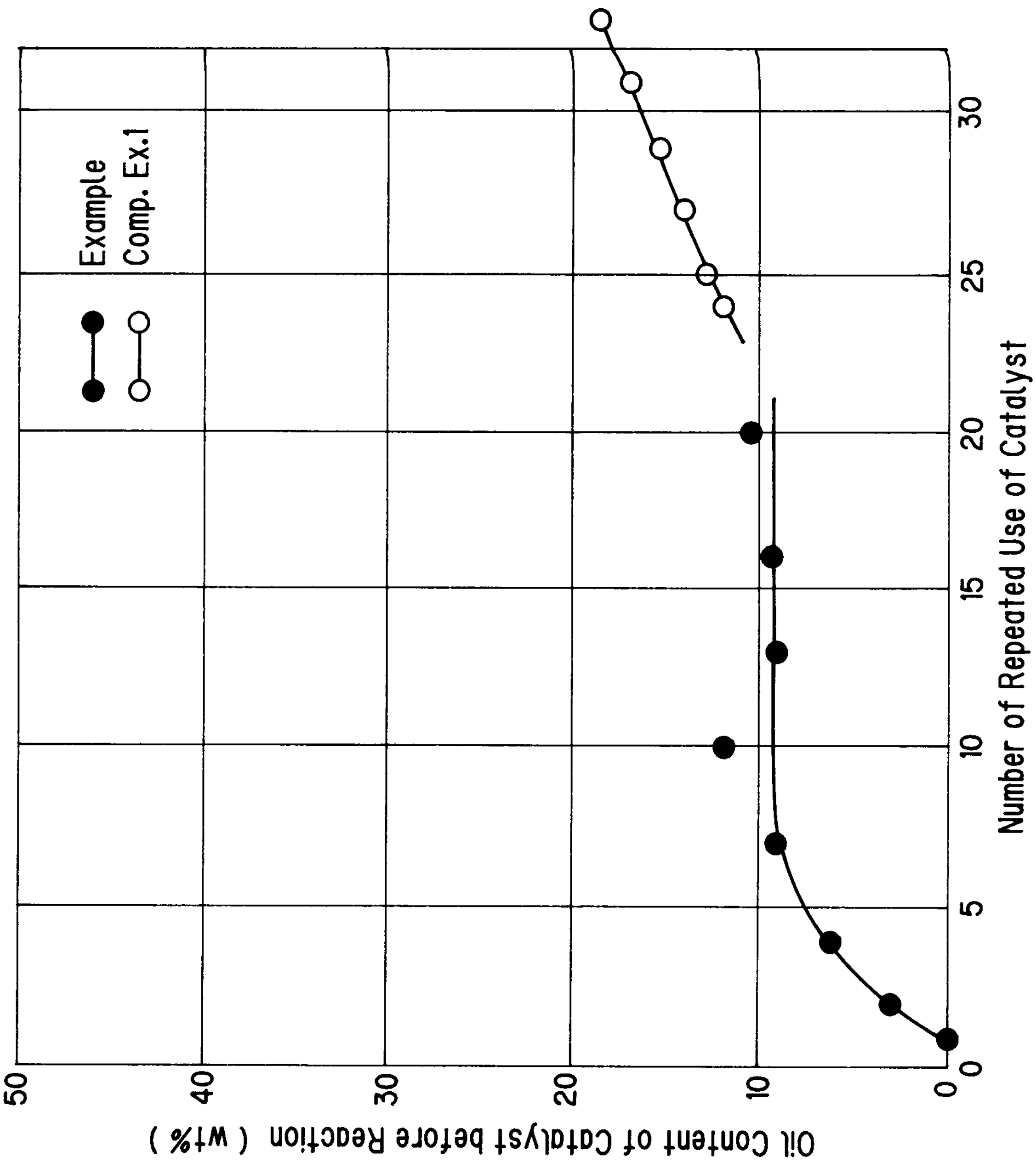
Example
Comp. Ex.1
Number of Repeated Use of Catalyst
Oil Content of Catalyst before Reaction ( wt% )
0
5
10
15
20
25
30
0
10
20
30
40
50

FIG. 4

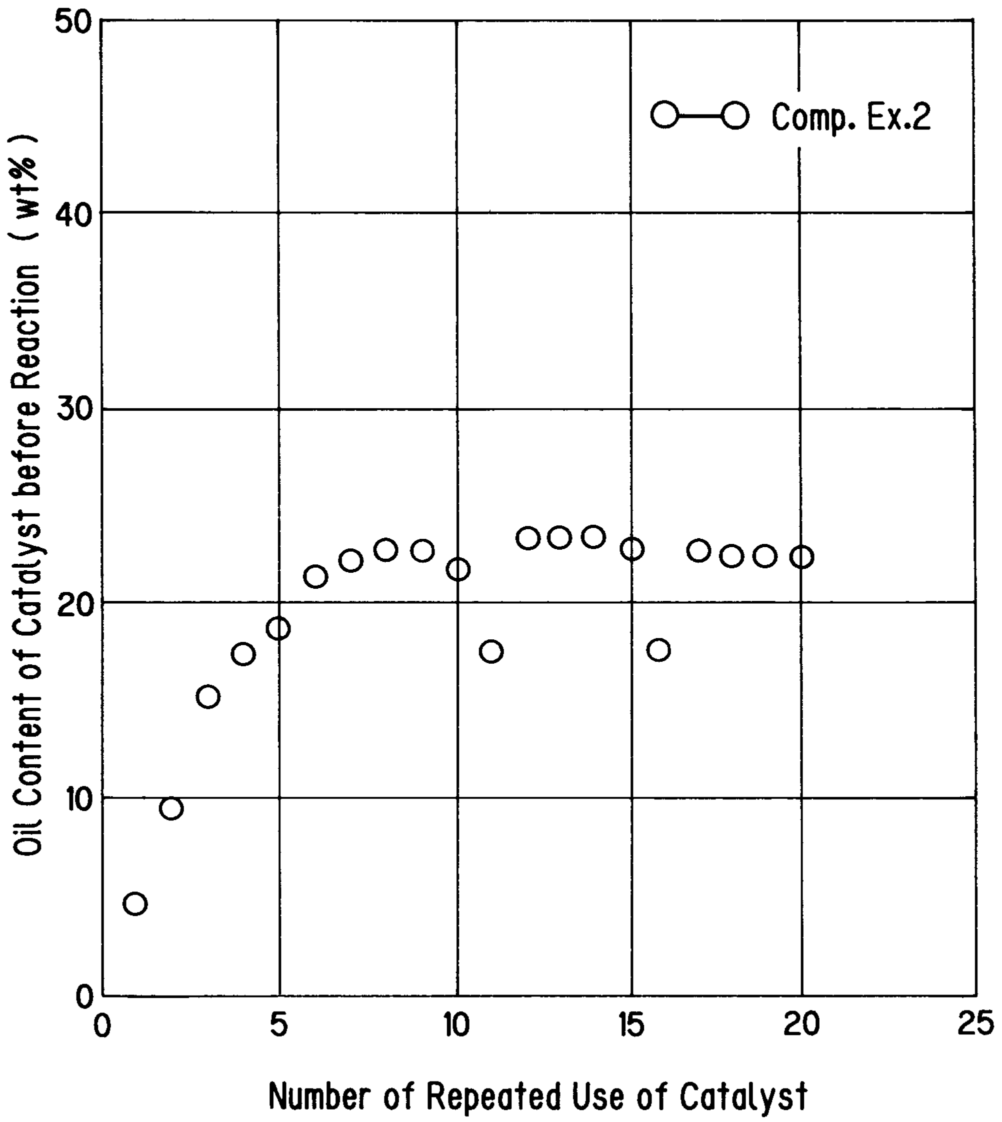
Oil Content of Catalyst before Reaction ( wt% )
0
10
20
30
40
50
0
5
10
15
20
25
Comp. Ex.2
Number of Repeated Use of Catalyst

FIG.5

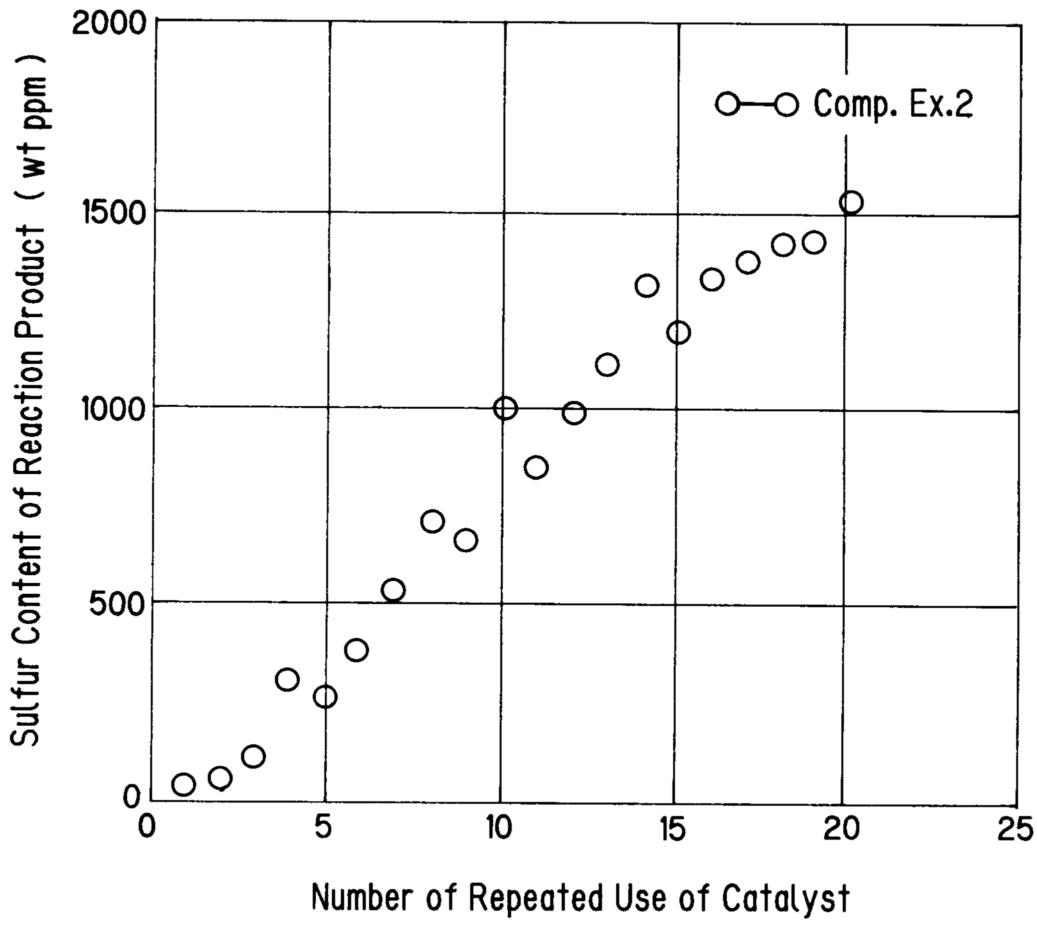
2000
1500
1000
500
0
0
5
10
15
20
25
Comp. Ex.2
Sulfur Content of Reaction Product ( wt ppm )
Number of Repeated Use of Catalyst